# EUROPÄISCHE PATENTSCHRIFT

(11) **EP 0 540 775 B1**
(45) Veröffentlichungstag und Bekanntmachung des Hinweises auf die Patenterteilung: **23.07.1997**
(21) Anmeldenummer: 91118994.2
(22) Anmeldetag: 07.11.1991
(51) Int. Cl.: A61M 11/06, A61M 15/00

(54) **Vernebler insbesondere zur Anwendung in Geräten für die Inhalationstherapie**
Atomiser particularly for inhalation therapy
Atomiseur en particulier pour la thérapie par inhalation

(43) Veröffentlichungstag der Anmeldung: 12.05.1993
(73) Patentinhaber: PAUL RITZAU PARI-WERK GmbH, D-82319 Starnberg (DE)
(72) Erfinder: Lintl, Andreas, W-8130 Starnberg (DE); Knoch, Martin, Dr.-Ing., W-8137 Berg (DE)
(74) Vertreter: Eitle, Werner, Dipl.-Ing.

(56) Entgegenhaltungen:
- EP-A- 0 052 284
- EP-A- 0 171 726
- EP-A- 0 261 649
- GB-A- 721 458

## Beschreibung

Die Erfindung betrifft einen Vernebler insbesondere zur Anwendung in Geräten für die Inhalationstherapie.

Nicht nur zur Behandlung von Atemwegserkrankungen, sondern in zunehmendem Maße auch zur Verabreichung anderer medizinischer Wirkstoffe wird die Inhalationstherapie angewandt. Bei dieser Therapieart wird der Wirkstoff in Form eines Flüssigkeitsnebels mit sehr kleinen röpfchendurchmessern (unter 5 µm) dem Patienten zur Inhalation dargeboten und zusammen mit der Atemluft in die Atemwege transportiert.

Zur Durchmischung von Atemluft und Flüssigkeitnebel sind in modernen Verneblern relativ großvolumige Verneblungsräume vorgesehen, die auch als Beruhigungskammern dienen. Damit für den Inhalationsvorgang eine ausreichende Menge des Flüssigkeitsnebels bereitgehalten wird, muß der Verneblungsraum relativ groß ausgelegt werden. Außerdem begünstigt ein großer Verneblungsraum die Durchmischung und Homogenisierung des Flüssigkeitsnebels. Die Größe des Verneblers beeinträchtigt jedoch auf der anderen Seite die Handhabung und Reinigungsmöglichkeiten. Ein weiterer Nachteil liegt darin, daß auch das Abscheiden bzw. Niederschlagen zu großer Flüssigkeitströpfchen nur in beschränktem Umfang erfolgt.

Aus EP-A-0 171 726 ist ein Vernebler bekannt, bei dem in einem Verneblungsraum mit Hilfe einer Venturi-Düse ein Aerosol erzeugt wird. Unmittelbar über der Venturi-Düse endet ein Zuluftkamin, der sich für die Zuführung von Umgebungsluft in den Verneblungsraum hinein erstreckt. Am unteren Ende besitzt der Zuluftkamin einen Prallschirm. Der Verneblungsraum wird durch eine Umlenkfläche in zwei Teile unterteilt. Das von der Düse erzeugte Aerosol strömt nach Durchmischung mit der durch den Kamin zugeführten Umgebungsluft an der Umlenkfläche entlang und durch einen ringförmigen Spalt um den Zuluftkamin, so daß das Aerosol von dem ersten in den zweiten Teil des Verneblungsraumes und von dort zum Ansaugstutzen gelangt.

Ausgehend von diesem Stand der Technik liegt der Erfindung die Aufgabe zugrunde, einen Vernebler anzugeben, der leicht zu zerlegen und zu reinigen ist und bei kompaktem Format einen sehr kleinen Tröpfchendurchmesser des Flüssigkeitsnebels erreicht.

Gelöst wird diese Aufgabe durch einen Vernebler mit den Merkmalen des Patentanpruchs 1. Vorteilhafte Ausgestaltungen ergeben sich aus den Unteransprüchen.

Wesentlich ist dabei, daß insbesondere der Prallabschnitt ein Mitreißen zu großer Flüssigkeitströpfchen in die Öffnung des Ansaugstutzens verhindert und für ein Abscheiden bzw. Niederschlagen der zu großen Flüssigkeitströpfchen sorgt, was letztlich zur Homogenisierung des Flüssigkeitsnebels führt. Daneben beeinflußt der Leitabschnitt unterstützend die Luftströmung im Verneblungsraum, da dadurch der Strömungsweg verlängert wird, um so eine bessere Durchmischung zu erreichen.

Im folgenden wird die Erfindung anhand von Ausführungsbeispielen unter Bezugnahme auf die Zeichnung genauer beschrieben, in der zeigt:
- Fig. 1: einen Vernebler mit integrierten Zerstäuber und dem erfindungsgemäßen Verneblungsraumteiler,
- Fig. 2A und 2B: den erfindungsgemäßen Verneblungsraumteiler an einem Bestandteil des Verneblers in unterschiedlichen Ansichten, und
- Fig. 3A bis 3F: verschiedene Ausgestaltungen des erfindungsgemäßen Verneblungsraumteilers.

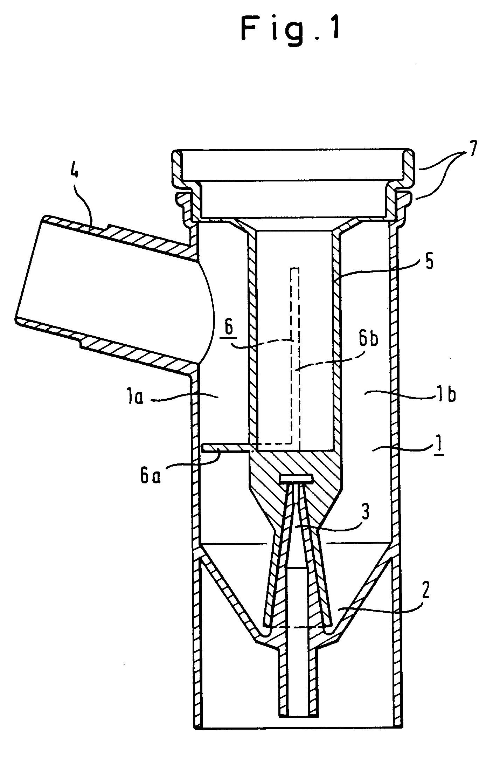

In Fig. 1 ist zur Erläuterung der Erfindung beispielhaft ein Vernebler mit integriertem Zerstäuber und mit einem im wesentlichen zylindrischen Verneblungsraum 1 dargestellt. Der Verneblungsraum 1 ist nach unten abgeschlossen durch einen Flüssigkeitssammelbereich 2, in den die Flüssigkeit eingefüllt wird, die mit Hilfe der Zerstäuberdüse 3 auf bekannte Weise derart zerstäubt wird, daß sich im Verneblungsraum 1 ein Flüssigkeitsnebel bildet. Der Flüssigkeitsnebel wird über einen Ansaugstutzen 4 aus dem Verneblungsraum 1 vom Patienten angesaugt. Um die dafür erforderliche Atemluftmenge zur Verfügung zu stellen, ist mittig im Verneblungsraum ein zylindrischer Zuluftkamin 5 vorgesehen, der an seinen Stirnseiten, d.h. nach oben und nach unten offen ist, so daß der weitaus größere Teil der Luft, die über den Ansaugstutzen 4 entnommen wird, durch den Zuluftkamin 5 in den Verneblungsraum 1 geführt wird. Das untere, in den Verneblungsraum ragende Ende des Zuluftkamins 5 liegt in unmittelbarer Nähe zum Zerstäubungsende der Zerstäuberdüse 3, um eine gute Homogenisierung des Flüssigkeitsnebels zu erreichen.

Der Vorgang des Durchmischens und Homogenisierens wird auf sehr effektive Weise erfindungsgemäß unterstützt durch den Verneblungsraumteiler 6, durch den ein Teil 1a des Verneblungsraumes 1, der in unmittelbarer Nähe zur Öffnung des Ansaugstutztens 4 liegt, in gewissem Umfang vom übrigen Teil 1b des Verneblungsraumes 1 abgeteilt wird. Die beiden Teile 1a und 1b sind aber so miteinander verbunden, daß weiterhin ein Ansaugen des Flüssigkeitsnebels aus dem Verneblungsraum 1 unter Zuführung von Atemluft über den Zufuhrkamin 5 möglich ist. Durch den erfindungsgemäßen Verneblungsraumteiler 6 wird aber eine Verbesserung der Homogenisierung des Flüssigkeitsnebels, sowie eine bessere Abscheidung zu großer Flüssigkeitströpfchen und eine verbesserte Mischung mit der zugeführten Aussenluft erreicht.

Der Verneblungsraumteiler 6 besteht aus mehreren Abschnitten, nämlich dem Prallabschnitt 6a und den Leitabschnitten 6b. Der Prallabschnitt 6a unterscheidet sich von den Leitabschnitten 6b hinsichtlich der Funktion. Trotz dieser Unterschiede, die im folgenden noch erläutert werden, kommt allen Abschnitten die oben angesprochene Abtrennfunktion zu, durch die die beiden Teile 1a und 1b des Verneblungsraumes 1 festgelegt werden.

Der Prallabschnitt 6a besitzt neben der Abtrennfunktion die Funktion einer Prallplatte, an der zu große Flüssigkeitströpfchen abgeschieden werden. Dadurch wird der vorteilhafte Effekt erreicht, daß zu große Flüssigkeitströpfchen nicht mehr oder in geringerem Maße über den Ansaugstutzen 4 angesaugt werden. Diese zu großen Flüssigkeitströpfchen gelangten bei herkömmlichen Verneblern aufgrund der kurzen Wegstrecke zwischen der Öffnung des Ansaugstutzens 4 und dem Zerstäubungsende der Zerstäuberdüse 3 in eine starke Strömung, die dazu führte, daß auch zu große Flüssigkeitströpfchen mitgerissen wurden. Der Prallabschnitt 6a des erfindungsgemäßen Verneblungsraumteilers 6 sorgt dafür, daß dieser kurze Weg unterbrochen ist oder daß die sich über diesen Weg ausbildende Strömung eine Behinderung erfährt.

Bereits durch den Prallabschnitt 6a sorgt der erfindungsgemäße Verneblungsraumteiler 6 für eine verbesserte Homogenisierung und eine Abscheidung zu großer Flüssigkeitströpfchen, die dann in den Flüssigkeitssammelraum 2 zurückgelangen, um erneut zerstäubt zu werden.

Verbessert wird diese Wirkung durch die Leitabschnitte 6b des erfindinngsgemäßen Verneblungsraumteilers 6. Die Leitabschnitte 6b verlaufen im wesentlichen senkrecht zum Prallabschnitt 6a und erstrecken sich in axialer Richtung des zylindrischen Verneblungsraums 1. Durch die Leitabschnitte 6b wird erreicht, daß die sich durch den Zuluftkamin 5 einstellende Luftströmung an den Leitabschnitten 6b entlang verläuft und über den Verbindungsbereich oberhalb der Leitabschnitte 6b zur Öffnung des Ansaugstutzens 4 verläuft. Dieser Verbindungsbereich verbindet die beiden Teile 1a und 1b des Verneblungsraumes 1 miteinander, so daß die erforderliche Luftströmung sich ausbilden kann. Die Leitabschnitte 6b des erfindungsgemäßen Verneblungsraumteilers 6 lenken die Luftströmung um und verlängern dadurch den Weg, so daß eine verbesserte Homogenisierung und Durchmischung des Flüssigkeitsnebels mit der angesaugten Luft, sowie eine verbesserte Abscheidung zu großer Flüssigkeitströpfchen erreicht wird.

Wie bereits zuvor erwähnt, ist bereits durch den Prallabschnitt 6a des erfindungsgemäßen Verneblungsraumteilers eine verbesserte Homogenisierung und Durchmischung des Flüssigkeitsnebels, sowie eine Abscheidung zu großer Flüssigkeitströpfchen erreichbar. Im Zusammenwirken mit dem Leitabschnitt 6b wird die Wirkung des erfindungsgemäßen Verneblungsraumteilers 6 noch weiter gesteigert.

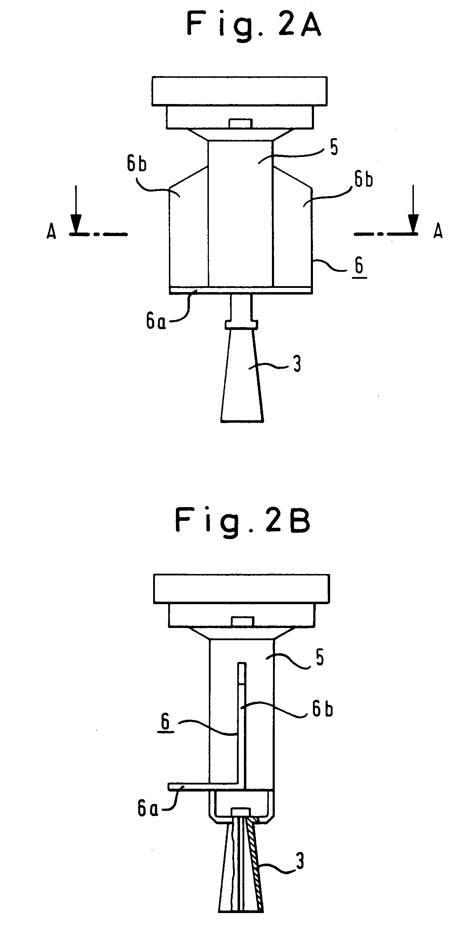

Im folgenden wird anhand der Figuren 2A und 2B die in Fig. 1 gezeigte Ausgestaltung des erfindungsgemäßen Verneblungsraumteilers genauer erläutert. Um den Aufbau des Verneblungsraumteilers zu verdeutlichen, wurde auf die Darstellung des den Verneblungsraum nach außen abschließenden Teils des Verneblers verzichtet und nur der Teil dargestellt, der den Zuluftkamin 5, den äußeren Teil der Zerstäuberdüse 3 und den erfindungsgemäßen Verneblungsraumteiler 6 bildet.

In Fig. 2A und 2B sind sowohl der Prallabschnitt 6a als auch die Leitabschnitte 6b des Verneblungsraumteilers 6 erkennbar. Alle Abschnitte erstrecken sich so weit, daß sie an die Innenwand des den Verneblungsraum 1 bildenden äußeren Teils des Verneblers heranreichen, wie für den Prallabschnitt 6a in Fig. 1 deutlich gezeigt ist. Ebenso erstrecken sich die Leitabschnitte 6b im wesentlichen bis zur Innenwand des Verneblungsraums. Auf diese Weise wird eine ausreichende Unterteilung des Verneblungsraumes 1 in einen ersten Teil 1a, der in unmittelbarer Nähe der Öffnung des Ansaugstutzens 4 liegt, und einen Teil 1b erreicht, der oberhalb des Flüssigkeitssammelraumes 2 liegt.

Wie aus den Fig. 2A und 2B hervorgeht, verlaufen die Leitabschnitte 6b radial zur Oberfläche des zylindrischen Zuluftkamins 5 und ferner in axialer Richtung dazu. Die oberen Enden der Leitabschnitte 6b, die nicht mit dem Prallabschnitt 6a verbunden sind, verlaufen vorteilhaft angeschrägt, wie sich aus Fig. 2A ergibt. Dadurch wird der Verbindungsquerschnitt, der die beiden Teile 1a und 1b des Verneblungsraumes 1 miteinander verbindet, für die Luftströmung vergrößert, ohne daß die Unterteilung aufgehoben wird. Da die Größe der Verbindungsquerschnitte oberhalb der Leitabschnitte 6b ebenfalls das Abscheideverhalten beeinflußt, kann über die Länge der Leitabschnitte 6b bzw. den Verlauf der oberen Kanten das Tröpfchenspektrum beeinflußt werden.

Der Prallabschnitt 6a verläuft senkrecht zu den Leitabschnitten 6b wie aus den Fig. 2A und 2B deutlich erkennbar ist. Der Prallabschnitt 6a wird letztlich gebildet durch einen Kreisringabschnitt, der senkrecht zur Längsachse der zylindrischen Zuluftkamins liegt, wie sich aus der folgenden Beschreibung spezieller Ausführungsformen des Prallabschnitts noch genauer ergibt.

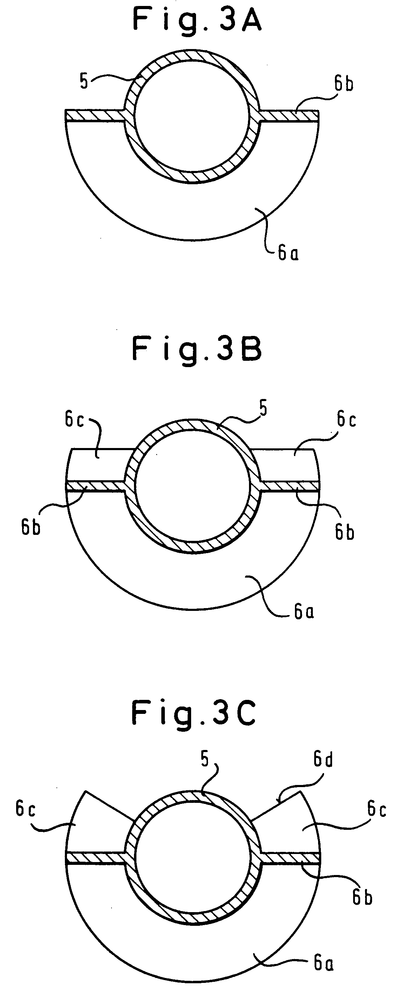

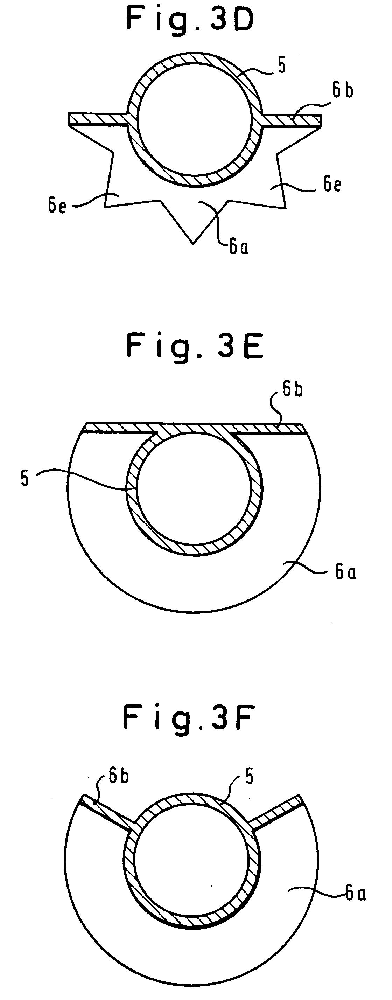

In den Fig. 3A bis 3F sind verschiedene Ausführungsbeispiele des Prallabschnitts 6a in einer Ansicht in Richtung des Schnitts A-A in Fig. 2A.

Fig. 3A zeigt die Grundform des erfindungsgemäßen Verneblungsraumteilers 6, der auch in den Fig. 1, 2A und 2B dargestellt ist. Deutlich erkennbar ist in Fig. 3A die Ausgestaltung des Prallabschnitts 6a als Teil eines Kreisringes, der sich von der Wandung des Zuluftkamins 5 radial nach außen und senkrecht zu den Leitabschnitten 6b erstreckt, die wiederum radial nach außen aber axial zum Zuluftkamin 5 verlaufen.

In Fig. 3B ist eine Ausführungsform dargestellt, bei der der Prallabschnitt 6a sich in dem Bereich hinter den Leitabschnitten 6b fortsetzt, in dem Ergänzungsabschnitte 6c vorgesehen sind. Durch die Ergänzungsabschnitte 6c des Prallabschnitts 6a wird eine verbesserte Abscheidung zu großer Flüssigkeitströpfchen erreicht, sowie eine verbesserte Durchmischung durch Wirbelbildung im Bereich oberhalb der Ergänzungsabschnitte 6c aufgrund der um die gerade Kante dieser Abschnitte herum beschleunigten Luftströmung.

Als besonders vorteilhaft hat sich eine Ausgestaltung der Ergänzungsabschnitte 6c erwiesen, bei der die Begrenzungskanten 6d der Ergänzungsabschnitte 6c radial verlaufen. Auch hier bildet sich eine stärker verwirbelte Strömung aus, die die Homogenisierung und Durchmischung des Füssigkeitsnebels mit der angesaugten Aussenluft verbessert. Ferner kann durch entsprechende Wahl des Öffnungswinkels zwischen den beiden Kanten 6d die Luftströmung beeinflußt werden, da die gesamte angesaugte Luftmenge durch diesen Abschnitt des Kreisringes hindurchtreten muß.

Bei der in Fig. 3D dargestellten Ausführungsform des Prallabschnitts 6a sind mehrere dreieckförmige Einschnitte vorgesehen, die dazu führen, daß bei dieser Ausführungsform der Prallabschnitt 6a im wesentlichen durch spitz zulaufende Flächenelemente 6e gebildet wird. Die Aussenkontur des Prallabschnitts 6a erhält dadurch einen gezackten Verlauf und bietet der Luftströmung Gelegenheit, durch die Ausschnitte hindurch zur Öffnung des Ansaugstutzens zu verlaufen. Jedoch sorgt der Prallabschnitt 6a auch in dieser Ausgestaltung für eine Behinderung der Luftströmung, so daß ein Mitreißen großer Flüssigkeitströpfchen wirksam vermieden werden kann. Ausschlaggebend ist dabei die Größe der spitz zulaufenden Flächen 6e bzw. der Ausschnitte an der Kontur des Prallabschnitts 6a.

Anstelle dreieckiger Ausschnitte können auch andere Formen aus der Kontur des Prallabschnitts 6a ausgeschnitten werden. Entscheidend ist, daß der Prallabschnitt 6a weiterhin seine die Luftströmung ausreichend behindernde Funktion erfüllt. In diesem Zusammenhang ist auch eine mit Löchern versehene Ausführungsform realisierbar, die eine unmittelbare Luftströmung zwischen dem Austrittsende der Zerstäuberdüse und dem Teil 1a des Verneblungsraumes zwar erlaubt, aber gleichzeitig ausreichend behindert, um eine Abscheidung zu großer Flüssigkeitströpfchen zu gewährleisten.

In Fig. 3E ist eine Ausführungsform gezeigt, bei der die Leitabschnitte soweit verschoben sind, daß sie tangential zum zylindrischen Körper des Zuluftkamins 5 verlaufen und einen einzigen Leitabschnitt 6b bilden. Der Prallabschnitt 6a erstreckt sich dementsprechend weit um den zylindrischen Zuluftkamin 5 herum und ist mit dem Leitabschnitt 6b verbunden. Durch diese Ausgestaltung wird die abscheidende Funktion des Prallabschnitts 6a erhöht, da die gesamte angesaugte Luftmenge jetzt durch einen verkleinerten Querschnitt hindurch an dem Leitabschnitt 6b vorbeiströmen muß.

Eine vergleichbare Wirkung hat die Ausführungsform gem. Fig. 3F, bei der sich ebenfalls der Prallabschnitt 6a weit um den Zuluftkamin 5 herum erstreckt. Jedoch sind bei dieser Ausführungsform die Leitabschnitte 6b radial so angeordnet, daß sich ihre Erstreckungsebenen unter einem Winkel ungleich 180° schneiden. Dieser Winkel kann, wie in Fig. 3F dargestellt ist, kleiner als 180°, aber auch größer als 180° sein. Bei einem Winkel kleiner als 180°, wie in Fig. 3F, wird der Strömungsquerschnitt für die angesaugte Luft verkleinert und somit die Strömungsgeschwindigkeit erhöht. Damit ergeben sich durch geeignete Wahl des Winkels Möglichkeiten zur Beeinflussung der Strömung und des Tröpfchenspektrums.

Alle Ausführungsbeispiele sind im wesentlichen symmetrisch zur Ebene des Schnitts in Fig. 1 aufgebaut, was auch auf die Anordnung des Ansaugstutzens 4 in Bezug auf den erfindungsgemäßen Verneblungsraumteiler 1 zutrifft. Jedoch ist es möglich, durch eine Verdrehung des den Verneblungsraumteiler tragenden Teiles, der in den Fig. 2A und 2B dargestellt ist, in Bezug auf den Teil des Verneblers, der den Ansaugstutzen 4 enthält, eine weitere Beeinflussung der Strömung zu erreichen. Insbesondere bei der Ausgestaltung des Verneblungsraumteilers, der einen Prallabschnitt 6a mit durchbrochener Aussenkontur gemäß Fig. 3D aufweist, läßt sich eine nahezu kontinuierliche Einstellung der Strömungsverhältnisse im Verneblungsraum erreichen, so daß eine Beeinflussung der Tröpfchengröße, der Strömungsgeschwindigkeiten, des Abscheideverhaltens an der Prallplatte und der Homogenisierung/Durchmischung möglich ist. Im oberen Bereich 7 des Verneblers gemäß Fig. 1 kann eine Skala angebracht werden, mit der diese Einstellung der Wirkung des Verneblers auf einfache Weise durchzuführen ist. Die Skala im Bereich 7 ist in Bezug auf die Lage des erfindungsgemäßen Verneblungsraumteilers 6 angebracht und berücksichtigt unter anderem die Art der zu zerstäubenden Flüssigkeit.

Die zu zerstäubende Flüssigkeit aber auch zahlreiche andere Größen sind bei der konkreten Ausgestaltung des erfindungsgemäßen Verneblungsraumteilers zu berücksichtigen. Je nach Anwendungsfall und Größe des Verneblers ist die eine oder andere Ausführungsform, etwa gemäß den Fig. 3A bis 3F zu bevorzugen, wobei auch eine Kombination der Einzelmerkmale dieser Ausführungsformen möglich ist. So kann zum Beispiel die Ausführungsform gemäß Fig. 3F mit einem Öffnungswinkel größer als 180° mit Ergänzungsabschnitten versehen werden, wie sie in den Fig. 3B und 3C dargestellt sind. Ferner kann eine durchbrochene Kontur, wie sie in Fig. 3D für den Prallabschnitt 6a dargestellt ist, auch bei den Prallabschnitten 6a der anderen dargestellten Ausführungsformen, sowie bei den Ergänzungsabschnitten 6c vorgesehen werden. Auch die Leitabschnitte 6b können mit durchbrochenen Konturen versehen werden.

## Patentansprüche

1. Vernebler zur Anwendung in Geräten für die Inhalationstherapie mit
- einem Verneblungsraum (1), der im wesentlichen zylinderförmig ausgestaltet ist und dem ein Flüssigkeitsnebel zugeführt oder in dem ein Flüssigkeitsnebel erzeugt wird,
- einem Ansaugstutzen (4) für die Entnahme des Flüssigkeitsnebels und
- einem Zuluftkamin (5) zur Zuführung von Umgebungsluft, der im wesentlichen zylindrisch ausgestaltet ist und sich in den Verneblungsraum (1) hinein erstreckt,
- einem Verneblungsraumteiler (6),
-- der in dem Verneblungsraum (1) derart angeordnet ist, daß der Verneblungsraum in einen ersten Teil (1a) in der Nähe des Ansaugstutzens (4) und einen zweiten Teil (1b) unterteilt ist, so daß die sich im Verneblungsraum einstellende Luft/Flüssigkeitnebel-Strömung beim Ansaugen des Flüssigkeitsnebels von dem zweiten Teil (1b) in den ersten Teil (1a) verläuft,
-- der integral mit dem Zuluftkamin (5) verbunden ist,
-- der einen Prallabschnitt (6a) umfaßt, der im kürzesten Weg zwischen dem Ansaugstutzen (4) und dem Ort des Zuführens oder Erzeugens des Flüssigkeitsnebels um den Zuluftkamin (5) herum angeordnet ist, so daß sich der Prallabschnitt im wesentlichen senkrecht zur Längsachse des Zuluftkamins erstreckt, und der eine Öffnung für die Luft/Flüssigkeitnebel-Strömung aufweist, und
-- der einen Leitabschnitt (6b) umfaßt, der mit dem Prallabschnitt (6a) integral ausgebildet ist und sich im wesentlichen in Richtung der Längsachse des Verneblungsraumes (1) erstreckt.

2. Vernebler nach Anspruch 1,
dadurch **gekennzeichnet,** daß
der Prallabschnitt (6a) kreisringförmig ist.

3. Vernebler nach Anspruch 2,
dadurch **gekennzeichnet,** daß
die Öffnung des Prallabschnitts (6a) einen Öffnungswinkel von 90° bis 270° aufweist.

4. Vernebler nach Anspruch 2,
dadurch **gekennzeichnet,** daß
die Öffnung des Prallabschnitts (6a) einen Öffnungswinkel von 180° aufweist.

5. Vernebler nach einem der Ansprüche 2 bis 4,
dadurch **gekennzeichnet,** daß
die Außenkontur des Prallabschnitts unterbrochen ist.

6. Vernebler nach Anspruch 5,
dadurch **gekennzeichnet,** daß
der Prallabschnitt (6a) zur Bildung einer unterbrochenen Außenkontur durch spitz zulaufende Flächenelemente (6e) gebildet ist.

7. Vernebler nach einem der Ansprüche 1 bis 6,
dadurch **gekennzeichnet,** daß
zwei Leitabschnitte (6b) vorgesehen sind.

8. Vernebler nach Anspruch 7,
dadurch **gekennzeichnet,** daß
die Leitabschnitte (6b) an den Kanten der Öffnung des Prallabschnitts (6a) angeordnet sind.

9. Vernebler nach Anspruch 8,
dadurch **gekennzeichnet,** daß
die Leitabschnitte (6b) an dem Prallabschnitt (6a) derart angeordnet sind, daß Ergänzungsabschnitte (6c) im zweiten Teil (1b) des Verneblungsraumes (1) gebildet werden.

10. Vernebler nach einem der Ansprüche 1 bis 9,
dadurch **gekennzeichnet,** daß
der Vernebler einen Bereich (7) zur Aufnahme einer Einstelleinrichtung aufweist, mit deren Hilfe über eine Verdrehung des Verneblungsraumteilers (6) im Verneblungsraum (1) eine Beeinflussung der sich einstellenden Luftströmung und des Flüssigkeitsnebels erfolgt.

## Claims

1. Nebulizer for use in apparatus for inhalation therapy, having
- a nebulizing chamber (1) which is essentially cylindrical in shape and to which is supplied a liquid mist or in which is produced a liquid mist,
- a suction pipe (4) for the removal of the liquid mist and
- an air supply flue (5), for supplying surrounding air, which is essentially cylindrical in shape and which projects into the nebulizing chamber (1),
- a nebulizing chamber divider (6),
- which is arranged in the nebulizing chamber (1) in such a way that the nebulizing chamber is sub-divided into a first part (1a) in the vicinity of the suction pipe (4) and into a second part (1b) so that the air/liquid mist appearing in the nebulizing chamber during the intake of the fluid mist flows from the second part (1b) into the first part (1a),
- which is integrally connected with the air supply flue (5),
- which comprises a baffle section (6a) which is arranged around the air supply flue (5) in the shortest path between the suction pipe (4) and the place where the liquid mist is supplied or where it is produced, so that the baffle sections extend essentially perpendicular to the longitudinal axis of the air supply flue and which has an opening for the air/liquid mist flow, and
- which comprises a guide section (6b) which is integrally constructed with the baffle section (6a) and which extends essentially in the direction of the longitudinal axis of the nebulizing chamber (1).

2. Nebulizer according to claim 1, characterised in that the baffle section (6a) has a circular annular shape.

3. Nebulizer according to claim 2, characterised in that the opening of the baffle section (6a) has an aperture angle of 90° to 270°.

4. Nebulizer according to claim 2, characterised in that the opening of the baffle section (6a) has an aperture angle of 180°.

5. Nebulizer according to one of the claims 2 to 4, characterised in that the outer contour of the baffle section is interrupted.

6. Nebulizer according to claim 5, characterised in that, for forming an interrupted outer contour, the baffle section (6a) is formed by flat elements (6e) meeting at a point.

7. Nebulizer according to one of the claims 1 to 6, characterised in that two guide sections (6b) are provided.

8. Nebulizer according to claim 7, characterised in that the guide sections (6b) are arranged on the edges of the opening of the baffle section (6a).

9. Nebulizer according to claim 8, characterised in that the guide sections (6b) of the baffle section (6a) are arranged in such a way that additional sections (6c) are formed in the second part (1b) of the nebulizing chamber (1).

10. Nebulizer according to one of the claims 1 to 9, characterised in that the nebulizer has an area (7) for receiving an adjusting device by means of which, by twisting the nebulizing chamber part (6), the air flow and the liquid mist appearing in the nebulizing chamber is influenced.

## Revendications

1. Atomiseur destiné à être utilisé dans des appareils pour le traitement par inhalation comportant
- une chambre 1 d'atomisation essentiellement cylindrique dans laquelle un brouillard de liquide est introduit ou produit,
- un conduit d'aspiration (4) pour le prélèvement du brouillard de liquide et
- un conduit 5 d'entrée d'air pour l'admission d'air ambiant, lequel conduit est sensiblement cylindrique et s'étend jusqu'à l'intérieur de la chambre d'atomisation (1),
- un dispositif (6) de séparation de la chambre d'atomisation
-- qui est disposé dans la chambre d'atomisation (1) de telle sorte que ladite chambre d'atomisation soit divisée en une première partie (1a) dans le voisinage du conduit d'aspiration (4) et une seconde partie (1b), de telle sorte que le courant d'air/brouillard de liquide qui s'établit dans la chambre d'atomisation lors de l'aspiration passe de la seconde partie (1b) dans la première partie (1a),
-- qui est lié d'une pièce au conduit d'entrée d'air (5),
-- qui comprend une partie brise-jet (6a) qui est disposée sur le trajet le plus court entre le conduit d'aspiration (4) et le point d'introduction ou de production du brouillard de liquide, autour du conduit d'entrée d'air (5), de telle sorte que la partie brise-jet s'étende essentiellement perpendiculairement à l'axe longitudinal du conduit d'entrée d'air, et qui présente une ouverture pour l'écoulement de l'air/du brouillard de liquide et
-- qui comprend une partie déflectrice (6b) qui est formée d'une pièce avec la partie brise-jet (6a) et s'étend essentiellement dans la direction de l'axe longitudinal de la chambre d'atomisation (1).

2. Atomiseur selon la revendication 1, caractérisé par le fait que la partie brise-jet (6a) a une forme d'anneau circulaire.

3. Atomiseur selon la revendication 2, caractérisé par le fait que l'ouverture dans la partie brise-jet (6a) présente un angle compris entre 90° et 270°.

4. Atomiseur selon la revendication 2, caractérisé par le fait que l'ouverture dans la partie brise-jet (6a) présente un angle de 180°.

5. Atomiseur selon l'une des revendications 2 à 4, caractérisé par le fait que le pourtour de la partie brise-jet (6a) est discontinu.

6. Atomiseur selon la revendication 5, caractérisé par le fait que la partie brise-jet (6a) aux de former un contour extérieur discontinu est constituée d'éléments de surface pointus.

7. Atomiseur selon une des revendications 1 à 6, caractérisé par le fait que deux parties déflectrices (6b) sont prévues.

8. Atomiseur selon la revendication 7, caractérisé par le fait que les parties déflectrices (6b) sont disposées sur les bords de l'ouverture de la partie brise-jet (6a).

9. Atomiseur selon la revendication 8, caractérisé par le fait que les parties déflectrices (6b) sont disposées sur les bords de l'ouverture de la partie brise-jet (6a) de telle sorte que des parties brise-jet (6c) complémentaires soient formées dans la seconde partie (1b) de la chambre d'atomisation (1).

10. Atomiseur selon une des revendications 1 à 9, caractérisé par le fait que l'atomiseur comporte une région (7) destinée à recevoir un dispositif de réglage à l'aide duquel on agit sur l'écoulement de l'air et du brouillard de liquide par une rotation du dispositif (6) de séparation de la chambre d'atomisation dans la chambre d'atomisation (1).
